# EUROPEAN PATENT APPLICATION

(11) **EP 3 190 412 A1**
(43) Date of publication of application: **12.07.2017**
(21) Application number: 15838552.6
(22) Date of filing: 31.08.2015
(51) Int. Cl.: G01N 33/50

(54) **REAGENT OR KIT FOR TESTING FOR CUTANEOUS LEISHMANIASIS, AND METHOD FOR TESTING FOR CUTANEOUS LEISHMANIASIS**

(30) Priority: 04.09.2014 JP 2014179912
(71) Applicant: The University of Tokyo, Tokyo 113-8654 (JP)
(72) Inventor: MATSUMOTO, Yoshitsugu, Tokyo 113-8654 (JP); GOTO, Yasuyuki, Tokyo 113-8654 (JP); SANJOBA, Chizu, Tokyo 113-8654 (JP); SUN, Jiamei, Tokyo 113-8654 (JP); OSADA, Yasutaka, Tokyo 113-8654 (JP)
(74) Representative: Graf von Stosch, Andreas
(86) International application number: PCT/JP2015/074756
(87) International publication number: WO 2016/035768

(57) **Abstract**

A reagent or kit for testing for cutaneous leishmaniasis, the reagent or kit including at least one of a crude leishmania protozoan antigen and a peroxiredoxin, wherein the reagent or kit is configured to be used to test for presence or absence of a type I allergic reaction by a skin test.

## Description

### Technical Field

The present invention relates to a reagent or kit for testing for cutaneous leishmaniasis and a method for testing for cutaneous leishmaniasis.

### Background Art

Leishmaniasis is an infectious disease caused by infection with leishmania protozoa. The leishmaniasis is spread over 88 countries in the tropical to subtropical regions and 350 million people are always exposed to a risk of infection therewith.

Morphologies of the leishmania protozoa include a promastigote with a flagellum and an amastigote without a flagellum. The leishmania protozoa are grown in the form of the promastigote in mid-guts of sand flies. The promastigotes invade vertebrate hosts through blood sucking by the sand flies, are transformed into amastigotes in macrophages, and proliferate. The hosts include human, dogs, and rodents. Therefore, the leishmaniasis is a zoonosis and an insect-borne infectious disease.

The leishmaniasis is classified roughly into cutaneous leishmaniasis and visceral leishmaniasis according to its disease type. One million to 1.5 million patients per year for the cutaneous leishmaniasis and 0.5 million patients per year for the visceral leishmaniasis are estimated to newly develop the diseases.

As common protozoa causing the cutaneous leishmaniasis, L. major complexes such as L. major are known for the cutaneous leishmaniasis in the Old World, while L. mexicana complexes such as L. mexicana are known for the cutaneous leishmaniasis in the New World.

In the cutaneous leishmaniasis, skin lesions are formed on portions through which sand flies sucked blood.

The cutaneous leishmaniasis does not cause death by itself, but is problematic in a wide distribution and the great number of patients.

There have been proposed anti-leishmania drugs useful as therapeutic drugs for leishmaniasis (for example, PTLs 1 and 2).

However, all of diagnosis, treatment, and prevention are important for controlling protozoan diseases. Therefore, at present, keen demand has arisen for rapidly providing a method for testing for leishmaniasis in which the leishmaniasis is able to be diagnosed easily, rapidly, and at a high precision using a less invasive means than blood collection.

### Citation List

### Patent Literature

PTL 1: International Publication No. WO2012/053408
PTL 2: International Publication No. WO2012/053232

### Summary of Invention

### Technical Problem

The present invention aims to solve the above existing problems and achieve the following object. That is, the present invention has an object to provide a reagent or kit for testing for cutaneous leishmaniasis in which the cutaneous leishmaniasis is able to be diagnosed easily, rapidly, and at a high precision using a less invasive means than blood collection, and a method for testing for cutaneous leishmaniasis using the reagent or kit for testing for cutaneous leishmaniasis.

### Solution to Problem

Means for solving the above problems are as follows.
<1> A reagent or kit for testing for cutaneous leishmaniasis, the reagent or kit including
   at least one of a crude leishmania protozoan antigen and a peroxiredoxin,
   wherein the reagent or kit is configured to be used to test for presence or absence of a type I allergic reaction by a skin test.
<2> A method for testing for cutaneous leishmaniasis, the method including:
   bringing at least one of a crude leishmania protozoan antigen and a peroxiredoxin into contact with a non-human animal skin using the reagent or kit for testing for cutaneous leishmaniasis according to <1>.

### Advantageous Effects of Invention

According to the present invention, it is possible to solve the above existing problems and achieve the above object. The present invention can provide a reagent or kit for testing for cutaneous leishmaniasis in which the cutaneous leishmaniasis is able to be diagnosed easily, rapidly, and at a high precision using a less invasive means than blood collection, and a method for testing for cutaneous leishmaniasis using the reagent or kit for testing for cutaneous leishmaniasis.

### Brief Description of Drawings

FIG. 1 is an image illustrating results of western blotting in Test Example 1.
FIG. 2 is a graph illustrating results of an ELISA assay in Test Example 2.
FIG. 3A is an image illustrating results in a rat which was inoculated with a crude leishmania protozoan antigen in Test Example 3.
FIG. 3B is an image illustrating results in a rat which was inoculated with a recombinant peroxiredoxin in Test Example 3.
FIG. 4A is an explanatory image of regions into which samples were inoculated in Test Example 4.
FIG. 4B is an image illustrating a mouse 5 minutes after Evans blue was inoculated thereinto in Test Example 4.
FIG. 4C is an image illustrating the reversed dorsal skin of a mouse in Test Example 4.

### Description of Embodiments

### (Reagent or kit for testing for cutaneous leishmaniasis)

A reagent or kit for testing for cutaneous leishmaniasis of the present invention includes at least one of a crude leishmania protozoan antigen and a peroxiredoxin; and, if necessary, further includes other components.

The reagent or kit for testing for cutaneous leishmaniasis is configured to be used to test for the presence or absence of a type I allergic reaction by a skin test.

The type I allergic reaction refers to an immediate allergic reaction in which IgE antibodies are involved.

### <Skin test>

The skin test is not particularly limited and may be appropriately selected from skin tests known in the art depending on the intended purpose, as long as it is able to test for the presence or absence of the type I allergic reaction. Examples thereof include skin patch tests (hereinafter may be referred to as "patch tests"), prick tests, scratch tests, and intracutaneous tests. Among them, patch tests are preferable from the viewpoint of easiness.

### <At least one of crude leishmania protozoan antigen and peroxiredoxin>

The crude leishmania protozoan antigen and the peroxiredoxin are used as antigens for testing whether a subject is infected with cutaneous leishmaniasis by the skin test.

The reagent or kit for testing for leishmaniasis may include the crude leishmania protozoan antigen alone, the peroxiredoxin alone, or both of the crude leishmania protozoan antigen and the peroxiredoxin.

### <<Crude leishmania protozoan antigen>>

The crude leishmania protozoan antigen includes a component derived from a leishmania protozoon.

A method for preparing the crude leishmania protozoan antigen is not particularly limited and may be appropriately selected depending on the intended purpose. For example, a frozen leishmania protozoon is subjected to freeze-thawing and then ultrasonic disruption. The resultant disrupted liquid is centrifuged. The resultant supernatant may be used as the crude leishmania protozoan antigen.

The leishmania protozoon is not particularly limited and may be appropriately selected depending on the intended purpose, as long as it causes the cutaneous leishmaniasis. Examples thereof include Leishmania major and Leishmania mexicana.

The leishmania protozoon may be in the form of a promastigote or an amastigote.

The number of times of the freeze-thawing is not particularly limited and may be appropriately selected depending on the intended purpose. For example, the number of times may be 6.

A condition for the ultrasonic disruption is not particularly limited and may be appropriately selected depending on the intended purpose. For example, a 10 second-ultrasonic disruption may be performed on ice every 10 seconds for 1 minute.

A condition for the centrifugation is not particularly limited and may be appropriately selected depending on the intended purpose. For example, the centrifugation may be performed at 10,000 × g for 30 minutes.

### <<Peroxiredoxin>>

The peroxiredoxin is a protein produced by the leishmania protozoon. The peroxiredoxin is an antigen useful for testing for the cutaneous leishmaniasis by the skin test because it causes a significant allergic reaction by itself, as demonstrated in the below-described Test Example.

The peroxiredoxin may be a purified product of peroxiredoxin produced by the leishmania protozoon or a recombinant peroxiredoxin. A recombinant peroxiredoxin is preferable from the viewpoint of safety.

A method for purifying the peroxiredoxin produced by the leishmania protozoon is not particularly limited and may be appropriately selected from purification methods known in the art.

A method for producing the recombinant peroxiredoxin is also not particularly limited and may be appropriately selected from methods known in the art. For example, the recombinant peroxiredoxin may be produced using E. coli into which a vector containing a nucleic acid coding for peroxiredoxin has been introduced, as described in the below-described Production Example.

Information about a base sequence coding for the peroxiredoxin produced by the leishmania protozoon is easily available from public databases.

For example, the base sequence coding for peroxiredoxin produced by L. major is deposited in GenBank (NCBI) under accession no.: XM_001681974.1 and the base sequence coding for peroxiredoxin produced by L. mexicana is deposited under accession no.: XM_003873581.1.

An amino acid sequence of the peroxiredoxin produced by the leishmania protozoon includes the amino acid sequence set forth in SEQ ID NO: 1.

The amino acid sequence of the peroxiredoxin may have a mutation anywhere therein, as long as effects of the present invention are not impaired. Examples of the mutation include deletion, substitution, and insertion.

The peroxiredoxin may consist of an epitope or may include the epitope repeatedly. The peroxiredoxin may be bound to a carrier protein.

Forms of the crude leishmania protozoan antigen and the peroxiredoxin are not particularly limited and may be appropriately selected depending on the intended purpose. For example, they may be in the form of an aqueous solution.

An amount of at least one of the crude leishmania protozoan antigen and the peroxiredoxin to be used in the skin test is not particularly limited and may be appropriately selected depending on the intended purpose.

### <Other components>

The other components are not particularly limited and may be appropriately selected depending on the intended purpose, as long as effects of the present invention are not impaired. Examples thereof include materials for the patch test, alcohols for skin-cleansing, physiological saline as a control, injection needles, needles for cotton threads, and instructions of the kit.

The materials for the patch test are not particularly limited and may be appropriately selected from those known in the art. Examples thereof include adhesive plasters.

### <Target>

A target species of the reagent or kit for testing for cutaneous leishmaniasis is not particularly limited and may be appropriately selected depending on the intended purpose. Examples thereof include human and dogs.

The reagent or kit for testing for cutaneous leishmaniasis is able to be suitably used for the below-described method for testing for cutaneous leishmaniasis of the present invention.

### (Method for testing for cutaneous leishmaniasis)

A method for testing for cutaneous leishmaniasis of the present invention includes using the reagent or kit for testing for cutaneous leishmaniasis of the present invention.

The method for testing for cutaneous leishmaniasis includes at least bringing at least one of a crude leishmania protozoan antigen and a peroxiredoxin into contact with a non-human animal skin (hereinafter may be referred to as "contact step"); and, if necessary, further includes other steps.

### <Contact step>

The contact step is a step of bringing at least one of a crude leishmania protozoan antigen and a peroxiredoxin into contact with a non-human animal skin.

### <<Non-human animal>>

The non-human animal is not particularly limited and may be appropriately selected depending on the intended purpose. Examples thereof include dogs.

### <<At least one of crude leishmania protozoan antigen and peroxiredoxin>>

The at least one of a crude leishmania protozoan antigen and a peroxiredoxin are the same as those described for the reagent or kit for testing for leishmaniasis of the present invention.

The method for bringing at least one of a crude leishmania protozoan antigen and a peroxiredoxin into contact with a non-human animal skin is not particularly limited and may be appropriately selected from skin test methods known in the art.

In the patch test, for example, an adhesive plaster onto which the at least one of a crude leishmania protozoan antigen and a peroxiredoxin have been dripped or applied may be applied to the skin.

In the prick test or the scratch test, for example, an injection needle or a needle for a cotton thread may be inserted under the skin, which has been cleansed with an alcohol-impregnated cotton ball, to the extent of slightly bleeding, followed by dripping a liquid containing the at least one of a crude leishmania protozoan antigen and a peroxiredoxin thereonto.

In the intracutaneous test, for example, a liquid containing the at least one of a crude leishmania protozoan antigen and a peroxiredoxin may be intracutaneously inoculated by injection.

A region with which the at least one of a crude leishmania protozoan antigen and a peroxiredoxin is brought into contact is not particularly limited and may be appropriately selected depending on the intended purpose. Examples thereof include the back, the ears, inside of the upper arms, and flexor of the forearm. The region may be a single region or a plurality of regions.

A period of time for which the at least one of a crude leishmania protozoan antigen and a peroxiredoxin are brought into contact with the skin is not particularly limited and may be appropriately selected depending on the intended purpose, as long as the presence or absence of the type I allergic reaction is able to be verified.

The presence or absence of the type I allergic reaction is usually able to be verified about 10 to 40 minutes after the at least one of a crude leishmania protozoan antigen and a peroxiredoxin are brought into contact with the skin.

### <Other steps>

The other steps are not particularly limited and may be appropriately selected depending on the intended purpose, as long as effects of the present invention are not impaired. However, the other steps preferably include an evaluation step.

### «Evaluation step»

The evaluation step is a step of testing whether there is a change in the skin after the contact step to thereby evaluate whether the skin is infected with leishmaniasis.

In the patch test, for example, the evaluation step may be performed according to the International Contact Dermatitis Research Group criteria. When a response occurred at a level equal to or lower than a certain level, it may be evaluated as negative (no infection with leishmaniasis). Meanwhile, when erythema or edema is observed on the region of the adhesive plaster onto which the at least one of a crude leishmania protozoan antigen and a peroxiredoxin have been dripped or applied, it may be evaluated as positive (infection with leishmaniasis). Note that, the certain level may be appropriately selected depending on test conditions.

In the prick test or the scratch test, for example, when a response occurred at a level equal to or lower than a certain level, it may be evaluated as negative. When a 15 mm or larger redness or a 4 mm or larger wheal is observed, it may be evaluated as positive. Note that, the certain level may be appropriately selected depending on test conditions.

In the intracutaneous test, for example, when a response occurred at a level equal to or lower than a certain level, it may be evaluated as negative. When a 20 mm or larger redness or a 9 mm or larger wheal is observed, it may be evaluated as positive. Note that, the certain level may be appropriately selected depending on test conditions.

Note that, a regent or kit for testing for cutaneous leishmaniasis of the present invention may also be used for a method for testing for cutaneous leishmaniasis targeting human. The method for testing for cutaneous leishmaniasis targeting human may be performed in the same manner as in the above-described method for testing for cutaneous leishmaniasis of the present invention, except that the non-human animal is changed to human.

A regent or kit for testing for cutaneous leishmaniasis of the present invention may also be used for supporting diagnosis of infection with cutaneous leishmaniasis in animals including human. That is, the present invention also relates to a method for supporting diagnosis of infection with cutaneous leishmaniasis in animals including human.

The method for supporting diagnosis of infection with cutaneous leishmaniasis in animals including human may be performed in the same manner as in the method for testing for cutaneous leishmaniasis of the present invention.

### Examples

The present invention will now be specifically described with reference to Test Examples and Production Examples described below, but the present invention is not limited thereto in any way.

### (Test Example 1: Examination of protozoan antigen recognized by IgE antibody)

### <Preparation of crude protozoan antigen>

A crude leishmania protozoan (L. major) antigen (hereinafter may be referred to as "SLA") was prepared as described below.

Promastigotes of Leishmania major were cultured in a flask, collected at the stationary phase from the flask, washed 3 times with PBS (-) (pH 7.2), suspended in 1 mL of PBS (-), and frozen at -80°C. This was repeatedly frozen and thawed 6 times. Then, the resultant suspension liquid was subjected to a 10 second-ultrasonic disruption on ice every 10 seconds for 1 minute using a BIORUPTURE ultrasonicator (available from Cosmo Bio Co., Ltd.). The resultant disruption liquid was placed together into a single tube and centrifuged at 10,000 × g for 30 minutes. The supernatant was collected and used as the crude leishmania protozoan antigen.

A protein concentration of the crude antigen was measured by a DC protein assay (available from Bio Rad) using bovine serum albumin (available from Sigma) as a standard. Then, the crude antigen was frozen and stored at -80°C.

### <SDS-PAGE and western-blotting>

The SLA was subjected to SDS-PAGE as described below.

The crude antigen was suspended in a SDS sample buffer (1% SDS, 5% mercaptoethanol, 10% glycerol, 0.05 M Tris-HCl pH 6.8, 10% bromophenol blue), heated at 100°C for 5 minutes, and then loaded on a 0.1% SDS/10% polyacrylamide gel at 10 µL per lane. The gel was subjected to electrophoresis at a constant current of 30 mA for 65 minutes using a BE-230 or BE-240 gel system (available from Bio-Craft).

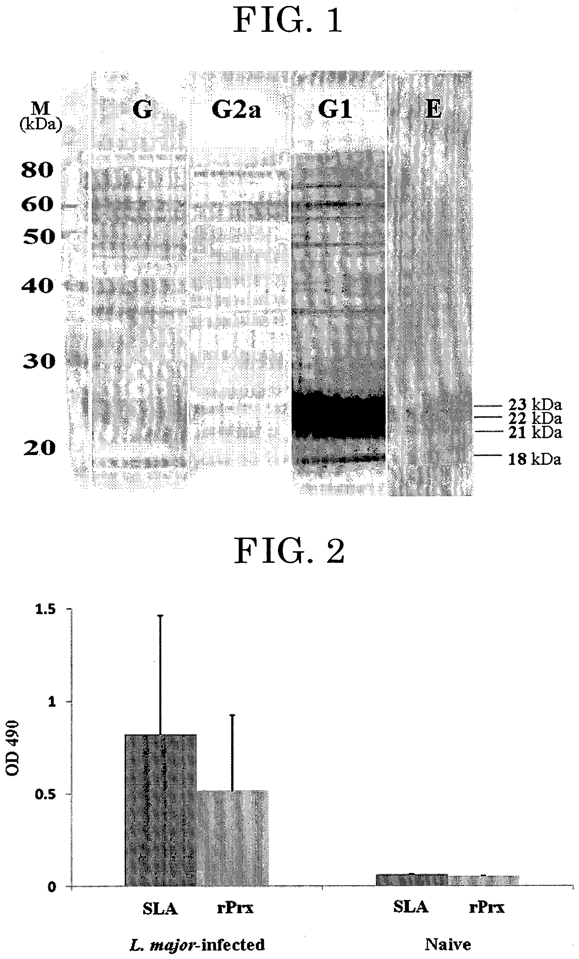

After the SDS-PAGE procedure, western-blotting was performed as described below. The results were presented in FIG. 1.

The gel, which had been subjected to the SDS-PAGE procedure, was transferred onto a PVDF membrane (HYBOND-PVDF, available from Amersham Pharmacia Biotech) at a constant current of 25 mA for 1 hour. Then, the membrane was left to stand at 4°C overnight while being immersed in 4% BLOCK ACE. Then, the membrane was washed 3 times with PBS-T for 10 minutes each and cut into strips.

A mouse serum sample was diluted 2,000 times (for IgG, IgG1, and IgG2a) or 500 times (for IgE) with PBS-T containing 0.4% BLOCK ACE.

After the strips of the membrane were left to stand in the diluted serums for 2 hours, the strips were washed 3 times with PBS-T for 10 minutes each. Detection was performed using a HRP-labeled goat anti-mouse IgG (γ chain-specific) antibody, an anti-IgG1 (γ1-specific) antibody, an anti-IgG2a (γ2a-specific) antibody, and an anti-IgE antibody (available from Southern Biotech. Assoc.). The anti-IgE antibody was diluted 1,000 times and other antibodies were diluted 5,000 times with PBS-T containing 0.4% BLOCK ACE.

The strips of the membrane were washed 3 times with PBS-T, visualized by an ECL detection system (available from GE Healthcare), and analyzed by LAS-3000 mini (available from FUJIFILM Corporation).

Note that, antibodies used in the western-blotting were as described below.

### [Primary antibody]

- BALB/c mouse serum 16 weeks after infection with L. major.

### [Secondary antibody]

- Anti-mouse IgG antibody (available from Southern Biotech (USA))
- Anti-mouse IgG1 antibody (available from Southern Biotech (USA))
- Anti-mouse IgG2a antibody (available from Southern Biotech (USA))
- Anti-mouse IgE antibody (available from Southern Biotech (USA))

In FIG. 1, M represents the maker, G represents the results in the case of using the anti-mouse IgG antibody as the secondary antibody, G2a represents the results in the case of using the anti-mouse IgG2a antibody as the secondary antibody, G1 represents the results in the case of using the anti-mouse IgG1 antibody as the secondary antibody, and E represents the results in the case of using the anti-mouse IgE antibody as the secondary antibody.

For the results in FIG. 1, in the case of using the anti-mouse IgE antibody as the secondary antibody, bands were detected at 18 kDa, 21 kDa, 22 kDa, and 23 kDa.

One band of the bands detected in the case of using the anti-mouse IgE antibody as the secondary antibody corresponded to the band with which a monoclonal antibody against a peroxiredoxin (hereinafter, may be referred to as "Prx"), which was a leishmania protozoan protein, was reacted.

### (Production Example 1: Production of recombinant peroxiredoxin)

The base sequence coding for the peroxiredoxin of Leishmania major (GenBank accession no.: XM_001681974.1) was obtained by PCR cloning using the below-described primers and L. major Friedlin genomic DNA as a template. The amplification product obtained by the PCR cloning was digested with NdeI and HindIII, and ligated into a pET-28a (+) vector (available from Novagen, USA). The insert was sequenced.

### [Primer]

Forward:
   5'-ttacatatgtcctgcggtaacgccaag-3' (SEQ ID NO: 2)
Reverse:
   5'-ttaaagcttttactgcttgctgaagtatc-3' (SEQ ID NO: 3)

In order to express a recombinant protein, the vector was transduced into E. coli ROSETTA™ competent cells (available from Novagen, USA). The E. coli was cultured in a SOB medium supplemented with 50 µg/mL of kanamycin and 34 µg/mL of chloramphenicol.

The recombinant protein was induced to be expressed by adding isopropyl β-D-1-thiogalactopyranoside (IPTG) thereto at a final concentration of 0.5 mM. Three hours after, the E. coli was collected through centrifugation at 2,000 g for 20 minutes.

The collected product (pellet) was suspended again in a lysis buffer (100mM Na₂HPO₄, 10mM Tris-HCL, pH 8.0) and lysed by ultrasonic treatment with BRANSON SONIFIER 250 (available from Branson, USA).

Then, the resultant was centrifuged at 10,000 g for 30 minutes. The recombinant protein dissolved in the supernatant was purified using a Ni-nitrilotriacetic acid (NTA) resin (available from Qiagen, USA) according to the protocol appended therewith. The purification with the Ni-NTA resin was repeated 4 times.

Then, the extracted fraction containing the recombinant protein was pooled and subjected to dialysis twice with 20 mM Tris-HCl (pH9.0). The dialyzed protein was incubated with a MACRO-PREP High Q support (available from Bio-Rad, USA) and then loaded onto a POLY-PREP chromatography column (available from Bio-Rad, USA). The recombinant protein was extracted with a concentration gradient of NaCl in Tris-HCl (pH9.0). The extracted fraction containing the recombinant protein was pooled and subjected to dialysis 4 times with 20 mM Tris-HCl (pH8.0). Then, the resultant was concentrated with an AMICON ultrafiltration device (available from Amicon, USA) and sterilized by passing through a MILLEX (registered trademark)-GP 0.22 µm Filter Unit (available from Millipore, USA).

A purity of the recombinant protein was evaluated by SDS-PAGE and Coomassie Blue staining. A concentration of the recombinant protein was measured by a DC protein assay (available from Bio-Rad (USA)) using bovine serum albumin as a standard. The recombinant protein was stored at -80°C.

Note that, the amino acid sequence of the recombinant protein (hereinafter may be referred to as "recombinant peroxiredoxin" or "rPrx") is presented in SEQ ID NO: 1.

### (Test Example 2: Detection of IgE antibody against rPrx)

An ELISA assay was performed as described below using, as an antigen, the crude leishmania protozoan (L. major) antigen (SLA) prepared in the same manner as in the Test Example 1 or the recombinant peroxiredoxin prepared in the Production Example 1 and, as a primary antibody, a BALB/c mouse serum 16 weeks after infection with L. major or a BALB/c mouse serum uninfected with L. major. The results are presented in FIG. 2.

One hundred microliters of a 0.1 M sodium carbonate buffer (pH9.6) containing 1 µg of the crude antigen or 200 ng of the recombinant peroxiredoxin was added per well to a 96-well plate (Nunc-immunoplate Maxisorp), which was left to stand at 4°C overnight. After removing the crude antigen or the recombinant peroxiredoxin, the plate was washed 3 times with a sodium phosphate buffer containing 0.05% PBS-T using a NUNC microplate washer. Two hundred microliters of PBS-T containing 1% BLOCK ACE was added to the plate, which was left to stand at room temperature for 1 hour.

One hundred microliters per well of the mouse serum sample, which had been diluted 100 times with PBS-T containing 0.4% BLOCK ACE, was added to the plate, which was left to stand at room temperature for 1 hour.

The plate was washed 3 times with PBS-T and then added with a HRP-labeled goat anti-mouse IgE antibody, which had been diluted 2,000 times with PBS-T containing 0.4% BLOCK ACE, as a secondary antibody. The plate was left to stand at room temperature for 1 hour.

In order to detect the IgE, 100 µL per well of o-phenylenediamine containing 0.01% hydrogen peroxide (1 mg/mL) was added to wells of the plate. The plate was left to stand in a light-shielded chamber at room temperature for 1 hour. Twenty microliters per well of 6 N sulfuric acid was added to the wells to stop the reaction. An ELISA microplate reader (available from BIO-RAD) was used to measure absorbance at 490 nm.

In FIG. 2, SLA represents the results in the case of using the crude leishmania protozoan antigen, rPrx represents the results in the case of using the recombinant peroxiredoxin, L. major-infected represents the results in the case of using the BALB/c mouse serum 16 weeks after infection with L. major, and Naive represents the results in the case of using the BALB/c mouse serum uninfected with L. major. Data are expressed as an average of OD values and error bars represent the standard deviations of OD values (n = 4).

For the results in FIG. 2, the IgE antibody against the recombinant peroxiredoxin was detected in the BALB/c mouse serum 16 weeks after infection with L. major.

### (Test Example 3: PCA (Passive cutaneous anaphylaxis) reaction)

A PCA reaction was used to verify whether an allergic reaction occurred in vivo due to a reaction between the crude leishmania protozoan antigen or the recombinant peroxiredoxin and the IgE antibody.

### -Sample-

The following samples were prepared:
- Crude leishmania protozoan (L. major) antigen (SLA) prepared in the same manner as in the Test Example 1;
- Recombinant peroxiredoxin produced in the Production Example 1;
- BALB/c mouse serum infected with L. major;
- BALB/c mouse serum uninfected with L. major;
- PBS (-) solution;
- C11C (monoclonal antibody against both of amastigotes and promastigotes of L. major, prepared at Laboratory of Molecular Immunology, Graduate School of Agricultural and Life Sciences, The University of Tokyo.);
- IgG antibody separated from the BALB/c mouse serum infected with L. major (prepared at Laboratory of Molecular Immunology, Graduate School of Agricultural and Life Sciences, The University of Tokyo.); and
- RAG2^{-/-} mouse (maintained and propagated at Laboratory of Molecular Immunology, Graduate School of Agricultural and Life Sciences, The University of Tokyo.).

Wister rats were intracutaneously inoculated with 100 µL of the BALB/c mouse serum infected with L. major (diluted twice, 10 times, 50 times, 250 times, 1,250 times, 6,250 times, 31,250 times, or 156,250 times), the BALB/c mouse serum uninfected with L. major (diluted twice, 10 times, 50 times, 250 times, 1,250 times, 6,250 times, or 31,250 times), the PBS (-) solution, the C11C (50 µg), the IgG antibody separated from the BALB/c mouse serum infected with L. major (100 µg), or the RAG2^{-/-} mouse serum.

Forty eight hours after the inoculation, the crude leishmania protozoan (L. major) antigen (1 mg) or the recombinant peroxiredoxin (200 µg) was inoculated into a base of the tail of each of the rats along with 1% by mass Evans blue (blue pigment).

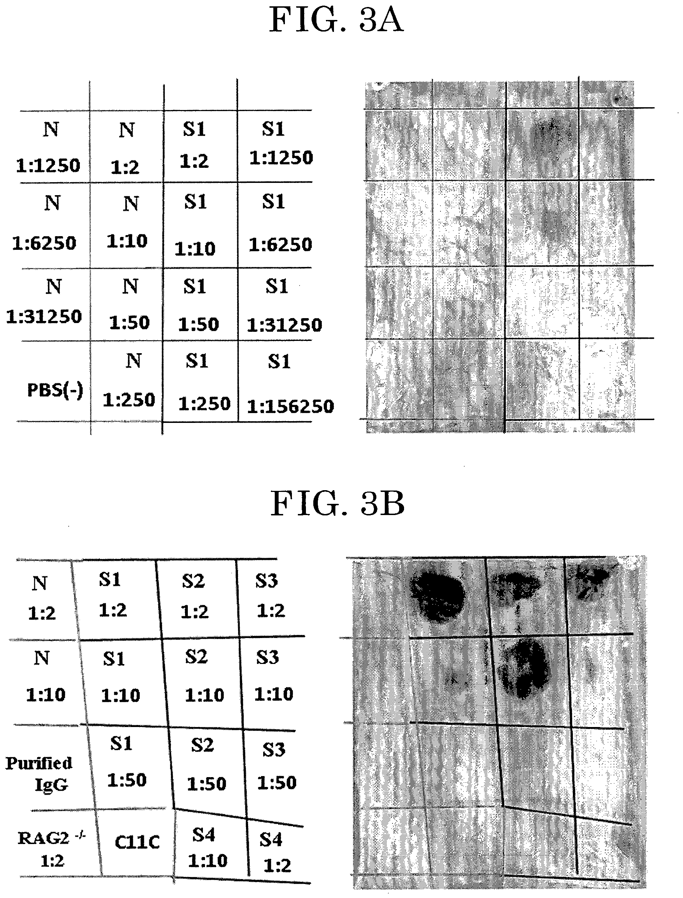

Thirty minutes after the inoculation, the rats were sacrificed. The dorsal skins were reversed and examined for anaphylaxis. The results are presented in FIGs. 3A and 3B.

FIG. 3A illustrates the results in the rat which was inoculated with the crude leishmania protozoan antigen. FIG. 3B illustrates the results in the rat which was inoculated with the recombinant peroxiredoxin.

In each of the figures, the figure on the right is an image illustrating the skin of the rat.

In each of the figures, the figure on the left is an explanatory image of regions in the figure on the right into which the BALB/c mouse serum infected with L. major, the BALB/c mouse serum uninfected with L. major, the PBS (-) solution, the C11C, the IgG antibody separated from the BALB/c mouse serum infected with L. major, or the RAG2^{-/-} mouse serum was inoculated. In each of the figures, N denotes the region into which the BALB/c mouse serum uninfected with L. major was inoculated; S1, S2, S3, and S4 denote the region into which the BALB/c mouse serum infected with L. major was inoculated; PBS (-) denotes the region into which the PBS (-) solution was inoculated; C11C denotes the region into which the C11C was inoculated; Purified IgG denotes the region into which the IgG antibody separated from the BALB/c mouse serum infected with L. major was inoculated; and RAG2^{-/-} denotes the region into which the RAG2^{-/-} mouse serum was inoculated. Moreover, 1:2 denotes 2 times dilution, 1:10 denotes 10 times dilution, 1:50 denotes 50 times dilution, 1:250 denotes 250 times dilution, 1:1250 denotes 1,250 times dilution, 1:6250 denotes 6,250 times dilution, 1:31250 denotes 31,250 times dilution, and 1:156250 denotes 156,250 times dilution.

For the results in FIGs. 3A and 3B, the region into which the BALB/c mouse serum infected with L. major was inoculated was stained blue and extravasation of Evans blue was observed. It is believed that this is because a reaction of the SLA or the recombinant peroxiredoxin with the IgE antibody, which was derived from the infected mouse serum and was bound to a surface of a mast cell, caused an allergic reaction to thereby increase vascular permeability. Note that, the allergic reaction had occurred 5 minutes after the inoculation of the crude leishmania protozoan (L. major) antigen or the recombinant peroxiredoxin.

### (Test Example 4: Allergic reaction)

It was revealed from the results of the Test Example 3 that the reaction of the crude leishmania protozoan antigen or the recombinant peroxiredoxin with the IgE antibody caused the allergic reaction. Next, it was examined whether inoculation with the crude leishmania protozoan antigen or the recombinant peroxiredoxin caused an allergic reaction in a mouse infected with L. major.

### -Sample-

The following samples were prepared:
- Crude leishmania protozoan (L. major) antigen (SLA) prepared in the same manner as in the Test Example 1;
- Recombinant peroxiredoxin produced in the Production Example 1;
- PBS solution; and
- BSA (available from Sigma).

The dorsal skin and the ears of a BALB/c mouse 10 weeks after infection with L. major were intracutaneously inoculated with the crude leishmania protozoan (L. major) antigen (dorsal skin: 50 µg, ear: 20 µg), the BSA (dorsal skin: 50 µg, ear: 20 µg), the recombinant peroxiredoxin (dorsal skin: 50 µg), or the PBS solution.

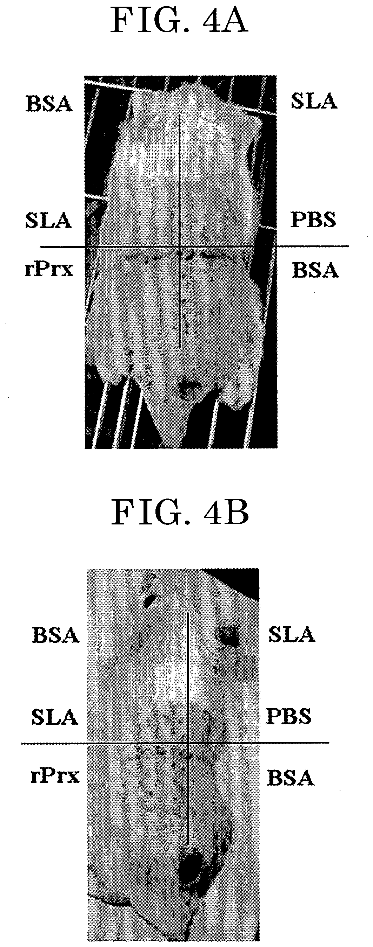

After the inoculation, 1% by mass Evans blue was inoculated into a base of the tail of the mouse. FIG. 4B illustrates the mouse 5 minutes after Evans blue was inoculated thereinto.

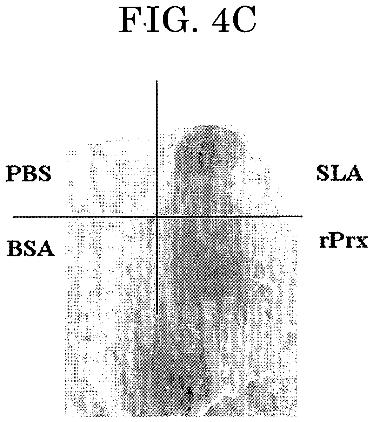

Thirty minutes after the inoculation of Evans blue, the mouse was sacrificed. The dorsal skin was reversed and examined for anaphylaxis. The results are presented in FIG 4C.

FIG. 4A illustrates the regions into which the crude leishmania protozoan (L. major) antigen, the BSA, the recombinant peroxiredoxin, and the PBS solution were inoculated.

In FIGs. 4A to 4C, BSA represents the region into which the BSA was inoculated, SLA represents the region into which the crude leishmania protozoan (L. major) antigen was inoculated, PBS represents the region into which the PBS was inoculated, and rPrx represents the region into which the recombinant peroxiredoxin was inoculated.

For the results in FIGs. 4B and 4C, likewise the crude leishmania protozoan antigen, the region into which the recombinant peroxiredoxin was inoculated was stained blue and extravasation of Evans blue was observed. Therefore, it was believed that, likewise the crude leishmania protozoan antigen, the recombinant peroxiredoxin also caused an allergic reaction.

Aspects of the present invention are as follows, for example:
<1> A reagent or kit for testing for cutaneous leishmaniasis, the reagent or kit including
   at least one of a crude leishmania protozoan antigen and a peroxiredoxin,
   wherein the reagent or kit is configured to be used to test for presence or absence of a type I allergic reaction by a skin test.
<2> The reagent or kit for testing for cutaneous leishmaniasis according to <1>, wherein the skin test is a patch test.
<3> The reagent or kit for testing for cutaneous leishmaniasis according to <1> or <2>, wherein the peroxiredoxin is a recombinant peroxiredoxin.
<4> A method for testing for cutaneous leishmaniasis, the method including:
   bringing at least one of a crude leishmania protozoan antigen and a peroxiredoxin into contact with a non-human animal skin using the reagent or kit for testing for cutaneous leishmaniasis according to any one of <1> to <3>.
<5> The method for testing for cutaneous leishmaniasis according to <4>, wherein the peroxiredoxin is a recombinant peroxiredoxin.

## Claims

1. A reagent or kit for testing for cutaneous leishmaniasis, the reagent or kit comprising
at least one of a crude leishmania protozoan antigen and a peroxiredoxin,
wherein the reagent or kit is configured to be used to test for presence or absence of a type I allergic reaction by a skin test.

2. The reagent or kit for testing for cutaneous leishmaniasis according to claim 1, wherein the skin test is a patch test.

3. The reagent or kit for testing for cutaneous leishmaniasis according to claim 1 or 2, wherein the peroxiredoxin is a recombinant peroxiredoxin.

4. A method for testing for cutaneous leishmaniasis, the method comprising:
bringing at least one of a crude leishmania protozoan antigen and a peroxiredoxin into contact with a non-human animal skin using the reagent or kit for testing for cutaneous leishmaniasis according to any one of claims 1 to 3.

5. The method for testing for cutaneous leishmaniasis according to claim 4, wherein the peroxiredoxin is a recombinant peroxiredoxin.
